(19) **Europäisches Patentamt / European Patent Office / Office européen des brevets**

(11) **EP 4 768 038 A1**

(12) **EUROPEAN PATENT APPLICATION**
published in accordance with Art. 153(4) EPC

(43) Date of publication:
01.07.2026 Bulletin 2026/27

(21) Application number: 24856730.7

(22) Date of filing: 12.08.2024

(51) International Patent Classification (IPC):
*A61K 38/08* (2019.01)       *A61P 3/04* (2006.01)
*A23L 33/18* (2016.01)

(52) Cooperative Patent Classification (CPC):
**A23L 33/18; A61K 38/08; A61P 3/04**

(86) International application number:
**PCT/KR2024/012000**

(87) International publication number:
**WO 2025/042118 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 24.08.2023 KR 20230111132

(71) Applicant: Ensol Biosciences Inc.
Daejeon 34036 (KR)

(72) Inventors:
• KIM, Hae Jin
Daejeon 34036 (KR)

• MOON, Eun Joung
Daejeon 34036 (KR)
• LEE, Young Hoon
Sejong 30064 (KR)

(74) Representative: **dompatent**
**Partnerschaft von Patentanwälten und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

(54) **NOVEL USE OF NONAPEPTIDE**

(57) The present invention provides a novel use of a peptide having an amino acid sequence of SEQ ID NO: 1 or a pharmaceutically acceptable salt thereof. The present invention has the advantage of effectively treating, preventing, improving, and/or suppressing one or more conditions selected from obesity and obesity-related complications.

[FIG. 1]

EP 4 768 038 A1

## Description

[Technical Field]

The present invention relates to a novel use of a nonapeptide, and more particularly, to a novel use of a peptide having an amino acid sequence of SEQ ID NO: 1 or a pharmaceutically acceptable salt thereof.

[Background Art]

Overweight and obesity are representative adult-onset metabolic disorders and are sometimes regarded as diseases (see Powell-Wiley TM, Poirier P, Burke LE, Després JP, Gordon-Larsen P, Lavie CJ, Lear SA, Ndumele CE, Neeland IJ, Sanders P, St-Onge MP. "Obesity and cardiovascular disease: A Scientific statement from the American Heart Association." Circulation. 2021 May 25;143(21):e984-e1010). With the increase in average life expectancy resulting from advances in medicine and improvements in living standards, and the decrease in physical activity due to the growth of the urban population, the prevalence of overweight and obesity has increased significantly. Obesity can cause various obesity-related complications, including hypertension, elevated LDL cholesterol, reduced HDL cholesterol, or high triglyceride levels (dyslipidemia), type 2 diabetes, coronary artery disease, stroke, gallbladder disease, osteoarthritis (degeneration of cartilage and bone within joints), sleep apnea and respiratory disorders, cancer, mental illnesses such as depression, anxiety, and other psychiatric disorders, as well as physical pain and impaired physical function (see Blüher M. "Obesity: global epidemiology and pathogenesis." Nat Rev Endocrinol. 2019 May;15(5):288-298; Whitlock G, Lewington S, Sherliker P, Clarke R, Emberson J, Halsey J, Qizilbash N, Collins R, Peto R. "Body-mass index and cause-specific mortality in 900,000 adults: Collaborative Analyses of 57 Prospective Studies." Lancet. 2009 Mar 28;373(9669):1083-96).

In addition, a study using mice with diet-induced obesity confirmed that weight loss through dietary control normalized glucose metabolism and promoted functional recovery from stroke induced by middle cerebral artery occlusion (see Karampatsi D, Zabala A, Wilhelmsson U, Dekens D, Vercalsteren E, Larsson M, Nyström T, Pekny M, Patrone C, Darsalia V. "Diet-induced weight loss in obese/diabetic mice normalizes glucose metabolism and promotes functional recovery after stroke." Cardiovasc Diabetol. 2021 Dec 22;20(1):240). Furthermore, clinical trials have demonstrated that weight reduction in overweight hypertensive patients effectively lowers blood pressure, indicating that weight loss directly contributes to the treatment and prevention of obesity-related complications (see Reisin E, Abel R, Modan M, Silverberg DS, Eliahou HE, Modan B. "Effect of weight loss without salt restriction on the reduction of blood pressure in overweight hypertensive patients." N Engl J Med. 1978 Jan 5;298(1):1-6).

Therefore, there is a need for the development of technologies capable of addressing overweight and obesity, as well as obesity-related complications.

Meanwhile, the present inventors developed a nonapeptide (a peptide having an amino acid sequence of SEQ ID NO: 1, which is an amino acid sequence consisting of nine amino acids, YGAGAGAGY, or a pharmaceutically acceptable salt thereof). The nonapeptide has been known to be effective in treating insulitis and type 1 diabetes (see International Patent Publication No. WO 2020/184901 A1). The inventors therefore made continuous efforts to discover a new use for this effective nonapeptide. However, when the nonapeptide was applied for the treatment of type 1 diabetes, some subjects showed an increase in body weight, and thus it was not predictable that the nonapeptide could be applied to obesity, which requires weight reduction.

[Prior Art Document]

[Patent Document]

(Patent Document 1) International Patent Application Publication No. WO 2020/184901 A1, September 17, 2020, Specification

[Non-Patent Document]

(Non-Patent Document 1) Powell-Wiley TM, Poirier P, Burke LE, Despres JP, Gordon-Larsen P, Lavie CJ, Lear SA, Ndumele CE, Neeland IJ, Sanders P, St-Onge MP. "Obesity and cardiovascular disease: A scientific statement from the American Heart Association." Circulation. 2021 May 25;143(21):e984-e1010
(Non-Patent Document 2) Blüher M. "Obesity: global epidemiology and pathogenesis." Nat Rev Endocrinol. 2019 May;15(5):288-298.
(Non-patent document 3) Whitlock G, Lewington S, Sherliker P, Clarke R, Emberson J, Halsey J, Qizilbash N, Collins R, Peto R. "Body-mass index and cause-specific mortality in 900,000 adults: collaborative analyses of 57 prospective studies." Lancet. 2009 Mar 28;373(9669):1083-96.

(Non-patent Document 4) Karampatsi D, Zabala A, Wilhelmsson U, Dekens D, Vercalsteren E, Larsson M, Nyström T, Pekny M, Patrone C, Darsalia V. "Diet-induced weight loss in obese/diabetic mice normalizes glucose metabolism and promotes functional recovery after stroke." Cardiovasc Diabetol. 2021 Dec 22;20(1):240.

(Non-patent Document 5) Reisin E, Abel R, Modan M, Silverberg DS, Eliahou HE, Modan B. "Effect of weight loss without salt restriction on the reduction of blood pressure in overweight hypertensive patients." N Engl J Med. 1978 Jan 5;298(1):1-6.

[Summary of Invention]

[Problems to be Solved by Invention]

An object of the present invention is to provide a novel use of a peptide having an amino acid sequence of SEQ ID NO: 1, or a pharmaceutically acceptable salt thereof.

The objects of the present invention are not limited to those described above, and other objects not specifically mentioned will be clearly understood by those skilled in the art from the following description.

[Means for Solving Problems]

Unexpectedly, the present inventors discovered that a peptide having an amino acid sequence of SEQ ID NO: 1 (YGAGAGAGY) or a pharmaceutically acceptable salt thereof (also referred to as a nonapeptide, "nona" meaning the number 9) is effective in treating obesity or obesity-related complications, thereby completing the present invention.

In the amino acid sequence, Y represents Tyrosine (Tyr), G represents Glycine (Gly), and A represents Alanine (Ala).

The amino acids constituting the peptide may be in L-, D-, or DL-forms, and the amino acids constituting the peptide of the present invention include all of these. In addition, it is also apparent that Y is to be interpreted as encompassing all amino acids referred to as Tyrosine or 4-hydroxyphenylalanine.

The peptide may include variants in which a portion of the peptide structure of the present invention is altered through natural or artificial mutation without affecting the primary activity.

The pharmaceutically acceptable salt may include, for example, hydrochloride, sulfate, phosphate, acetate, citrate, tartrate, succinate, lactate, maleate, fumarate, oxalate, methanesulfonate, trifluoromethanesulfonate, benzenesulfonate, p-toluenesulfonate, sodium salt, potassium salt, calcium salt, and the like.

The present invention provides a pharmaceutical and/or food use of a peptide having an amino acid sequence of SEQ ID NO: 1 or a pharmaceutically acceptable salt thereof for the treatment, prevention, improvement, and/or suppression of one or more conditions selected from obesity and obesity-related complications. The term "treatment" encompasses the improvement or alleviation of symptoms associated with conditions selected from obesity and obesity-related complications, and the term "prevention" encompasses the suppression of progression from a pre-disease stage to a disease state.

In one embodiment, the present invention provides a pharmaceutical composition for the treatment or prevention of one or more conditions selected from obesity and obesity-related complications, including a peptide having an amino acid sequence of SEQ ID NO: 1 or a pharmaceutically acceptable salt thereof as an active ingredient.

The obesity includes overweight.

The obesity-related complications are not limited as long as they are related to obesity, but may preferably one or more selected from the group consisting of hypertension, elevated LDL cholesterol, reduced HDL cholesterol, and high triglyceride levels (dyslipidemia); type 2 diabetes; coronary artery disease; stroke; gallbladder disease; osteoarthritis (degeneration of cartilage and bone within joints); sleep apnea; respiratory disorders; cancer; depression; anxiety; mental illnesses; physical pain; impaired physical function; glucose metabolism disorders; stroke (preferably stroke induced by middle cerebral artery occlusion); and hypertension (preferably hypertension in an overweight hypertensive patient). This is because it is widely known that such obesity-related complications can be treated, prevented, improved, and/or suppressed by treating, preventing, improving, and/or suppressing obesity (including being overweight) (see Blüher M. "Obesity: global epidemiology and pathogenesis." Nat Rev Endocrinol. 2019 May;15(5):288-298; Whitlock G, Lewington S, Sherliker P, Clarke R, Emberson J, Halsey J, Qizilbash N, Collins R, Peto R. "Body-mass index and cause-specific mortality in 900,000 adults: collaborative analyses of 57 prospective studies." Lancet. 2009 Mar 28;373(9669):1083-96; Karampatsi D, Zabala A, Wilhelmsson U, Dekens D, Vercalsteren E, Larsson M, Nyström T, Pekny M, Patrone C, Darsalia V. "Diet-induced weight loss in obese/diabetic mice normalizes glucose metabolism and promotes functional recovery after stroke." Cardiovasc Diabetol. 2021 Dec 22;20(1):240; Reisin E, Abel R, Modan M, Silverberg DS, Eliahou HE, Modan B. "Effect of weight loss without salt restriction on the reduction of blood pressure in overweight hypertensive patients." N Engl J Med. 1978 Jan 5;298(1):1-6).

In addition, the composition may further include a pharmaceutically acceptable additive, and may be formed of the peptide or a pharmaceutically acceptable salt thereof and the additive.

The peptide may be prepared by a method commonly used in the field of peptide synthesis. For example, the peptide may be prepared by well-known methods such as solution-phase synthesis or solid-phase synthesis.

Examples of methods for forming peptide bonds include methods for activating carboxylic acid groups, such as the acyl azide method, acyl halide method, acyl imidazole method, carbodiimide method, phosphonium method, anhydride method, and mixed anhydride method; a method of dehydration condensation using a direct condensing agent; an oxidation-reduction method; and a method using Woodward's reagent K.

Prior to performing the condensation reaction, carboxyl groups, amino groups, and the like that are not involved in the reaction may be protected, and carboxyl groups involved in the condensation reaction may be activated by methods known in the art.

Examples of protecting groups for a carboxyl group may include ester-forming groups such as methyl, t-butyl, aryl, pentafluorophenyl, benzyl, p-methoxybenzyl, or methoxyethoxymethyl.

Examples of protecting groups for an amino group may include tritylcarbonyl, aryloxycarbonyl, cyclohexyloxycarbonyl, trichloroethyloxycarbonyl, benzyloxycarbonyl, t-butoxycarbonyl, and/or 9-fluorenylmethyloxycarbonyl.

Examples of activated forms of a carboxyl group may include mixed anhydrides, azides, acyl chlorides, or activated esters, such as esters formed with alcohols (e.g., pentachlorophenol, 2,4-dinitrophenol, cyanomethyl alcohol, p-nitrophenol, N-hydroxy-5-norbornene-2,3-dicarboxylimide, N-hydroxysuccinimide, N-hydroxyphthalamide, or 1-hydroxybenzotriazole).

Solvents usable in the condensation reaction for forming peptide bonds may include benzene, toluene, hexane, acetone, nitromethane, cyclohexane, ether, chloroform, dichloromethane, ethyl acetate, N,N-dimethylformamide, dimethyl sulfoxide, pyridine, dioxane, tetrahydrofuran, water, methanol, or ethanol, either as a single solvent or as a mixed solvent thereof.

The reaction temperature generally used may range from about -70 °C to 100 °C, and more preferably -30 °C to 30 °C.

The reaction for removing protecting groups from the peptide may vary depending on the type of protecting group, but the protecting group may be removed using oxidizing compounds, basic compounds, or transition metals capable of detaching the protecting group without affecting the peptide bond.

The protecting group may be removed by acid treatment, for example, using hydrogen chloride, hydrogen bromide, hydrogen fluoride, acetic acid, methanesulfonic acid, trifluoromethanesulfonic acid, trifluoroacetic acid, trimethylchlorosilane, or a mixture thereof.

When performing a reaction for removing the protecting group by the acid treatment, the reaction may be promoted by adding an auxiliary agent such as anisole, phenol, or thioanisole.

The protecting group may be removed by base treatment, for example, using ammonia, diethylamine, hydrazine, morpholine, N-methylpyrrolidine, piperidine, sodium carbonate, or a mixture of these bases.

In addition, the protecting group may be removed by transition metal treatment, for example, using zinc, mercury, or palladium/hydrogen.

After completion of the reaction, the peptide may be purified by conventional peptide purification methods, such as extraction, phase separation, solid precipitation, recrystallization, or column chromatography.

In addition, a peptide composed of the amino acid sequence of SEQ ID NO: 1 may be converted into a variant or a pharmaceutically acceptable salt thereof by conventional methods.

The peptide may be synthesized using an automated peptide synthesizer or produced by genetic engineering techniques. For example, a fusion gene encoding a fusion protein composed of a fusion partner and the peptide of the present invention may be constructed through genetic engineering, the host microorganism may be transformed with the fusion gene, and the fusion protein may then be expressed in the host microorganism. Subsequently, the peptide of the present invention may be cleaved and separated from the fusion protein using a protease or a chemical compound to produce the desired peptide.

The peptide or a pharmaceutically acceptable salt thereof may be administered primarily by parenteral routes, for example, by intravenous or subcutaneous injection, intrathecal administration, transdermal administration, nasal administration, or rectal administration. In addition, oral administration may also be possible in some cases.

The peptide or a pharmaceutically acceptable salt thereof, or the composition, may be formulated together with pharmaceutically acceptable additives into dosage forms such as injections, suppositories, powders, nasal drops, granules, tablets, or transdermal patches.

The pharmaceutically acceptable additives may be selected and added based on various factors well known to those skilled in the art, including, but not limited to, the specific bioactive substance employed, its concentration, stability, and intended bioavailability; the disease or condition to be treated; the age, size, and general condition of the subject to be treated; and the route of administration of the composition, such as nasal, oral, ocular, topical, transdermal, or intramuscular. Pharmaceutically acceptable additives commonly used for administering physiologically active substances by non-oral routes may include D5W (5% glucose in water), dextrose, and aqueous solutions containing physiological saline up to 5% by volume. In the case of local injection into a lesion, various injectable hydrogels may be used to enhance therapeutic efficacy and prolong the duration of action. In addition, the pharmaceutically acceptable additives may include additional components that enhance the stability of active ingredients, such as preservatives and antioxidants. The peptide of the present invention, or a pharmaceutically acceptable salt thereof, or the composition, may be formulated by any suitable method known in the art, and reference may be made to widely known literature in the field.

The peptide may be stored in a physiological saline solution and, after the addition of mannitol or sorbitol, may be lyophilized in ampoules. When used for administration, it may be dissolved in physiological saline or the like.

In addition, the present invention provides a food composition for improving or preventing one or more conditions selected from obesity and obesity-related complications, including a peptide having an amino acid sequence of SEQ ID NO: 1 or a pharmaceutically acceptable salt thereof as an active ingredient.

The food composition may be variously incorporated into foods, including beverages, and may be in the form of beverages, gum, tea, or health functional foods. The health functional foods may be formulated into dosage forms such as tablets or capsules. The term "health functional food" refers to a food manufactured (including processing; the same applies hereinafter) using raw materials or ingredients having beneficial functionality for the human body, as defined in the Health Functional Foods Act of the Republic of Korea. The term "functionality" refers to obtaining beneficial effects for health purposes, such as regulating nutrients or exerting physiological actions on the structure or function of the human body.

In addition to the active ingredient, the food composition may further include other components as essential ingredients without particular limitation. For example, the food composition may include various flavoring agents or natural carbohydrates as additional ingredients, similar to those in ordinary beverages. Examples of the above-described natural carbohydrates may include common sugars such as monosaccharides, for example, glucose, fructose, etc.; disaccharides, for example maltose, sucrose, etc.; and polysaccharides, for example, dextrin and cyclodextrin, as well as sugar alcohols such as xylitol, sorbitol, or erythritol, etc. In addition to the above-described flavoring agents, natural flavorings (thaumatin, stevia extracts (e.g., rebaudioside A, glycyrrhizin, etc.)) and synthetic flavorings (e.g., saccharin, aspartame, etc.) may be advantageously used. The proportion of the natural carbohydrates may be appropriately determined by those skilled in the art. In addition to the components, the food composition may include various nutrients, vitamins, minerals (electrolytes), flavoring agents such as synthetic flavorings and natural flavorings, coloring agents and thickeners (e.g., cheese, chocolate, etc.), pectic acid and salts thereof, alginic acid and salts thereof, and organic acids, protective colloidal thickeners, pH adjusters, stabilizers, preservatives, glycerin, alcohol, carbonation agents used in carbonated beverages, etc. These components may be used independently or in combination, and their proportions may be appropriately selected by those skilled in the art.

Unless otherwise specified, the same contents described for the pharmaceutical composition of the present invention shall apply equally to the food composition of the present invention, insofar as they are not inconsistent therewith. The term "improvement" is encompassed within "treatment" and refers to the alleviation or amelioration of a condition or symptom. The term "prevention" includes "suppression," and the term "suppression" refers to inhibiting the occurrence of a condition or symptom.

The peptide having an amino acid sequence of SEQ ID NO: 1 or a pharmaceutically acceptable salt thereof may be formulated by adding pharmaceutically or food-acceptable additives, such as carriers, excipients, or diluents. Reference for formulation may be made to widely known literature in the field.

Therefore, the composition of the present invention may further include a pharmaceutically acceptable additive or a food-acceptable additive, and may be formed of the active ingredient and the pharmaceutically acceptable additive or food-acceptable additive.

The composition of the present invention may include the active ingredient in an amount of 0.01 to 99.99% by weight.

The present invention also provides a method for treating, preventing, improving or suppressing one or more conditions selected from obesity and obesity-related complications, including administering a peptide having an amino acid sequence of SEQ ID NO: 1 or a pharmaceutically acceptable salt thereof to an animal (including a human or a non-human animal).

The animal may be a mammal.

The animal may be one in need of administration of a peptide having an amino acid sequence of SEQ ID NO: 1 or a pharmaceutically acceptable salt thereof.

The animal may be one suffering from, or at risk of developing, one or more conditions selected from obesity and obesity-related complications.

In addition, the peptide having an amino acid sequence of SEQ ID NO: 1 or a pharmaceutically acceptable salt thereof to be administered may be in an effective amount of the peptide having an amino acid sequence of SEQ ID NO: 1 or a pharmaceutically acceptable salt thereof.

Furthermore, the present invention provides the use of a peptide having an amino acid sequence of SEQ ID NO: 1 or a pharmaceutically acceptable salt thereof for preparing a formulation for improving, suppressing, treating, and/or preventing one or more conditions selected from obesity and obesity-related complications. The formulation may be a pharmaceutical formulation and/or a food formulation.

Unless otherwise specified, the contents described for the pharmaceutical compositions, food compositions, methods, and uses of the present invention shall apply equally to each other insofar as they are not inconsistent therewith.

The dosage of the peptide or a pharmaceutically acceptable salt thereof included in the compositions, uses, or methods of the present invention, or administered parenterally, may range from 0.0243 mg/day to 4860 mg/day, and preferably from 0.0486 mg/day to 2430 mg/day. For oral administration, the dosage may be 5 to 10 times that of the parenteral dosage. Administration may be carried out once daily or in several divided doses. Although the above dosage is based on an adult (weighing 60 kg), it will be understood that the dosage may vary depending on body weight, physical condition, and other factors. Meanwhile, when administered to a non-human animal, the dosage may vary depending on the species according to the calculation of the human equivalent dose (HED) based on the widely known body-surface-area conversion method (see USFDA, Guidance for Industry: Estimating the Maximum Safe Starting Dose in Adult Healthy Volunteers, Rockville, MD: US Food and Drug Administration; 2005).

[Advantageous effects]

According to the present invention, one or more conditions selected from obesity and obesity-related complications can be effectively treated, prevented, improved, and/or suppressed.

[Brief Description of Drawings]

FIG. 1 is a graph showing the weight-loss effect over time.
FIG. 2 is a graph showing the weight-loss effect by experimental group.

[Mode for Carrying out Invention]

Hereinafter, the advantages and features of the present invention, and methods for achieving them, will become apparent with reference to the examples described in detail below together with the accompanying drawings. However, the present invention is not limited to the examples set forth below, but may be embodied in various different forms. These examples are provided only to ensure a full disclosure of the present invention and to fully convey the scope of the invention to those skilled in the art. The present invention is defined solely by the scope of the claims.

Throughout the specification, the term "and/or" encompasses each and every possible combination of one or more of the mentioned components.

The terminology used herein is intended for the purpose of describing examples and is not intended to limit the present invention. In the specification, the singular form also includes the plural form unless otherwise specifically stated. The terms "includes" and/or "including" as used herein do not exclude the presence or addition of one or more other components and/or steps.

Hereinafter, in the examples, it was confirmed using obesity-induced experimental animals that a peptide having an amino acid sequence of SEQ ID NO: 1 or a salt thereof exhibits efficacy against obesity and obesity-related complications.

The reagents used in the examples were of the highest commercially available grade, and were purchased from Sigma and other suppliers.

<Example 1> Confirmation of the effects of the peptide on the improvement, treatment, suppression, and/or prevention of obesity and obesity-related complications

1-1. Preparation of peptide

The peptide listed in Table 1 below was prepared using a solid-phase peptide synthesis method. Specifically, the synthesis was carried out by a solid-phase method utilizing the chemical properties of Fmoc (9-fluorenylmethoxycarbonyl). More specifically, 0.55 mmol/g of solid-phase resin (Wang resin; Sigma-Aldrich), 5 mL of dimethylformamide (DMF), 1.1 mmol of Fmoc-Tyr(tBu)-OH, and 0.55 mmol of O-benzotriazole-N,N,N',N'-tetramethyl-uronium-hexafluoro-phosphate (HBTU) were added to a well-dried reactor and stirred at room temperature for 2 hours to synthesize Fmoc-Tyr(tBu)-resin. The synthesized resin was filtered and washed with dimethylformamide. Then, 8 mL of a 20% piperidine solution (in dimethylformamide) was added to the washed resin and stirred at room temperature for 30 minutes, thus synthesizing a Fmoc (fluorenylmethyloxycarbonyl protecting group)-deprotected Tyr(tBu)-resin. The synthesized Tyr(t-Bu)-resin was filtered and washed with dimethylformamide.

To the washed Tyr(tBu)-resin, 5 mL of dimethylformamide, 1.1 mmol of Fmoc-Gly-OH, and 0.55 mmol of O-benzotriazole-N,N,N',N'-tetramethyl-uronium-hexafluorophosphate (HBTU) were added and stirred at room temperature for 2 hours to synthesize Fmoc-Gly-Tyr(tBu)-resin. The synthesized resin was filtered and washed with dimethylformamide. Then, 8 mL of a 20% piperidine solution (in dimethylformamide) was added to the washed resin and stirred at room temperature for 30 minutes, thus synthesizing a Fmoc-deprotected Gly-Tyr(tBu)-resin. The synthesized Gly-Tyr(tBu)-resin was filtered and washed with dimethylformamide. Through this process, a peptide bond between glycine and tyrosine was formed.

Subsequently, Fmoc-Ala-OH, Fmoc-Gly-OH, Fmoc-Ala-OH, Fmoc-Gly-OH, Fmoc-Ala-OH, Fmoc-Gly-OH, and Fmoc-Tyr(tBu)-OH were sequentially used, and the same procedure as the peptide bond formation process between glycine and tyrosine was repeated to prepare a peptide-resin compound having the amino acid sequence listed in Table 1.

Then, 10 mL of a 95:5 (v/v) mixture of trifluoroacetic acid and water was added, stirred at room temperature for 3 hours, and filtered. Diethyl ether was added to the resulting filtrate to crystallize the solid. The resulting solid was filtered, washed with diethyl ether, and dried to obtain a crude peptide compound having the amino acid sequence listed in Table 1.

The crude peptide compound was purified by reverse-phase high-performance liquid chromatography (RP-HPLC) using a Shimadzu 5 mm Shim-pack ODS C18 column (20 $\times$ 250 mm) and lyophilized to obtain the peptide of the example as a white solid.

The purified peptide was analyzed by analytical reverse-phase high-performance liquid chromatography (analytical RP-HPLC) using a Shim-pack 5 mm ODS C18 column (4.6 $\times$ 250 mm), and its molecular weight was confirmed using a matrix-assisted laser desorption/ionization mass spectrometer (MALDI-MS) (Axima CFR, Kratos Analytical, Manchester, UK).

[TABLE 1]

|  | Amino acid sequence | Sequence No. |
|---|---|---|
| Example 1 | YGAGAGAGY | 1 |

In Table 1, Y represents Tyrosine (Tyr), G represents Glycine (Gly), and A represents Alanine (Ala).

1-2. Confirmation of the inhibitory effect on weight gain in obesity-induced animals

It was experimentally confirmed that the peptide prepared in section 1-1 inhibited weight gain in obesity-induced animals.

The experimental animals used were 6-week-old male C57BL/6 mice and db/db mice (BKS.Cg-m +/+ Leprdb/J, hereinafter referred to as db/db mice) (Jackson Laboratory). The C57BL/6 mice, which do not develop obesity, were used as the normal group in this experiment. The db/db mice, which develop obesity due to a mutation in the leptin receptor, are widely used in obesity research. These mice exhibit hyperphagia, obesity, hyperinsulinemia, and hyperglycemia, and obesity appears from 3 to 4 weeks of age. Animal breeding was conducted after approval from the Ensol Biosciences Institutional Animal Care and Use Committee (ES-2023-004). An acclimation period of one week was allowed, and the light/dark cycle was maintained at 12 hours (lights on at 08:00 and off at 20:00). The temperature was maintained at 20 $\pm$ 2°C, the relative humidity at 60 to 80%, and the experimental animals were fed a standard diet.

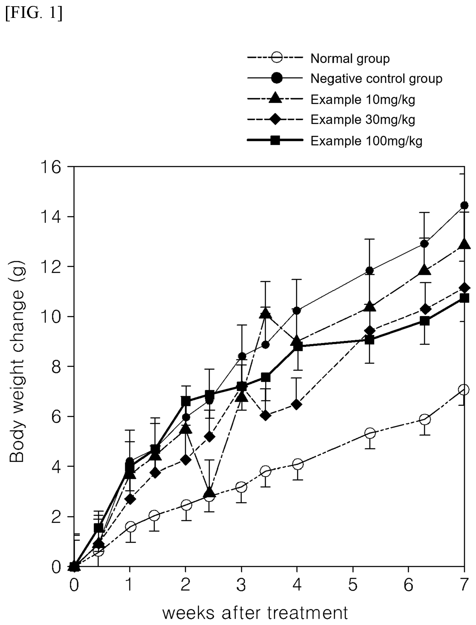

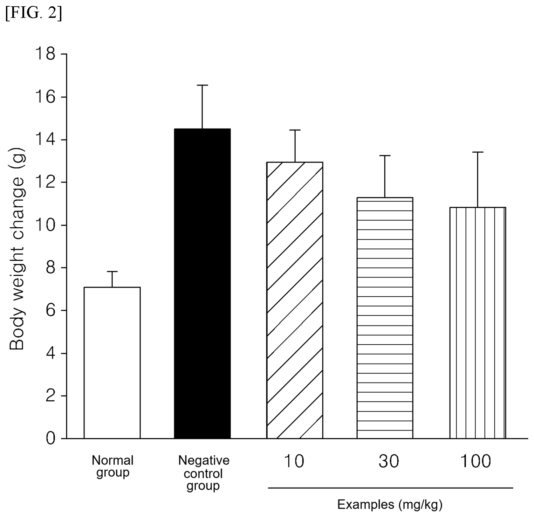

After a one-week acclimation period, the body weights of all experimental animals were measured and the experiment was conducted. The peptides (10, 30, and 100 mg/kg) prepared in section 1-1 were dissolved in phosphate-buffered saline (PBS) and orally administered to db/db mice at a dose of 200 $\mu$L per mouse using an oral gavage device. Mice in the normal and negative control groups received PBS (phosphate-buffered saline, Welgene) instead of the peptides prepared in section 1-1. Administration was performed twice daily, in the morning and evening, five consecutive days per week for four weeks, for a total of 40 doses. Body weights were measured twice weekly. After completion of the four-week administration, the body weights were further measured for an additional three weeks to observe the persistence of the anti-obesity effect. Each group, including the normal group, consisted of five mice. The observed changes in body weight are shown in FIGS. 1 and 2.

FIGS. 1 and 2 are graphs illustrating the results of analyzing the effects of the peptides (Example) prepared in section 1-1 on the inhibition or reduction of weight gain. Specifically, FIG. 1 is a graph showing the weight-loss effect over time, and FIG. 2 is a graph showing the weight-loss effect by experimental group.

The x-axis of FIG. 1 represents time (weeks) from the start of administration of the Example peptide, and the y-axis represents the increase in body weight (g) relative to that before the start of administration. In FIG. 2, the x-axis represents the experimental groups, and the y-axis represents the increase in body weight (g) relative to that before the start of administration.

First, as shown in FIG. 1, the body weight of mice in the normal group steadily increased over time during the 7-week period, reaching an increase of 4.1 g at the end of Example administration (week 4) and 7.1 g after 7 weeks. The negative control group of db/db mice exhibited a faster weight gain than the normal group, showing an increase of 10.3 g at the end of 4 weeks of administration and 14.5 g after 7 weeks. This corresponds to approximately 2- to 2.5-fold greater weight gain compared to the normal group, confirming the suitability of the db/db mice as an obesity animal model. The mice administered 10, 30, and 100 mg/kg of the Example peptide gained 9.1, 6.5, and 8.8 g, respectively, at the end of the 4-week administration period, confirming the weight gain-suppressing effect of oral nonapeptide administration.

As shown in FIG. 2, 3 weeks after completion of administration, i.e., at week 7, the body weight increases were 13.0, 11.2, and 10.8 g, respectively. This indicates that even after discontinuation of nonapeptide administration, body weight did not increase as rapidly as that in the negative control.

In addition, the results of the changes in body weight three weeks after completion of sample administration for each experimental group, compared to the increase observed in the negative control group, are shown in Table 2 below. For the normal group and the Example administration groups, the weight gain reduction rate was calculated according to Equation 1 below.

[Equation 1] Weight gain reduction rate (%) = (Weight gain in negative control group - Weight gain in normal group or Example administration group) / Weight gain in negative control group $\times$ 100 [Equation 1]

[TABLE 2]

| Group | Weight gain reduction rate (%) (Compared to the weight gain of the negative control group) |
| --- | --- |
| Normal group | 50.6 |
| Example 10 mg/kg | 10.4 |
| Example 30 mg/kg | 22.5 |
| Example 100 mg/kg | 25.4 |

As shown in Table 2, the normal group exhibited a 50.6% reduction in body weight compared to the negative control group. In addition, the mice administered 10, 30, and 100 mg/kg of the Example peptide showed weight reduction rates of 10.4%, 22.5%, and 25.4%, respectively. Although these rates were lower than that of the normal group, each showed dose-dependent weight reduction effect compared to the negative control group. These results indicate that oral administration of the nonapeptide suppressed weight gain in db/db mice, an obesity animal model. Therefore, the peptide of the present invention has effects of improving, treating, suppressing, and/or preventing obesity and obesity-related complications.

Meanwhile, the phenomenon in which body weight increased upon administration of the nonapeptide to type 1 diabetic animals may be interpreted as a recovery of the body weight that had been reduced due to type 1 diabetes, resulting from the therapeutic effect of the nonapeptide on type 1 diabetes.

<Preparative Example 1> Preparation of powder

    500 mg of the peptide prepared in the same manner as in Example 1-1
    100 mg of lactose monohydrate
    10 mg of talc
    The above ingredients were mixed and filled into an airtight pouch to obtain a powder preparation.

<Preparative Example 2> Preparation of tablets

    500 mg of the peptide prepared in the same manner as in Example 1-1
    100 mg of corn starch

100 mg of lactose monohydrate
2 mg of magnesium stearate
The above ingredients were mixed and compressed into tablets according to a conventional tablet manufacturing method.

<Preparative Example 3> Preparation of capsules

500 mg of prepared in the same manner as in Example 1-1
3 mg of crystalline cellulose
14.8 mg of lactose
0.2 mg of magnesium stearate
The above ingredients were mixed and filled into gelatin capsules according to a conventional capsule manufacturing method to obtain a capsule preparation.

<Preparative Example 4> Preparation of injection

500 mg of prepared in the same manner as in Example 1-1
180 mg of mannitol
2,793 mg of sterile distilled water for injection
26 mg of sodium dihydrogen phosphate

The above ingredients were prepared per ampoule (2 mL) according to a conventional injection preparation method.

<Preparation Example 5> Preparation of liquid preparation

500 mg of the peptide prepared in the same manner as in Example 1-1
10 g of isomerized sugar
5 g of mannitol
An appropriate amount of purified water
An appropriate amount of lemon flavor

Each ingredient was dissolved in purified water according to a conventional method for preparing liquid preparations, and an appropriate amount of lemon flavor was added. Purified water was then added to adjust the total volume to 100 mL, and the solution was filled into brown bottles to obtain a liquid preparation.

<Preparative Example 6> Manufacture of health functional food

1 mg of the peptide prepared in the same manner as in Example 1-1
An appropriate amount of a vitamin mixture
70 $\mu$g of Vitamin A acetate
1.0 mg of Vitamin E
0.13 mg of Vitamin B1
0.15 mg of Vitamin B2
0.5 mg of Vitamin B6
0.2 $\mu$g of Vitamin B12
10 mg of Vitamin C
10 $\mu$g of biotin
1.7 mg of nicotinamide
50 $\mu$g of folic acid

0.5 mg of calcium pantothenate
an appropriate amount of a mineral mixture
1.75 mg of ferrous sulfate
0.82 mg of zinc oxide
25.3 mg of magnesium carbonate
15 mg of monopotassium phosphate
55 mg of dicalcium phosphate
90 mg of potassium citrate
100 mg of calcium carbonate
24.8 mg of magnesium chloride

The composition ratio of the vitamin and mineral mixtures described above represents a preferred example of ingredients suitable for health foods. However, the mixing ratio may be arbitrarily modified as needed. The above ingredients were mixed according to a conventional method for manufacturing health functional foods, and granules were prepared. The resulting granules may be used to manufacture a health functional food composition according to a conventional method.

<Preparative Example 7> Manufacture of health drink

0.1 mg of the peptide prepared in the same manner as in Example 1-1
15 g of Vitamin C
100 g of Vitamin E (powder)
19.75 g of ferrous lactate
3.5 g of zinc oxide
3.5 g of nicotinamide
0.2 g of Vitamin A
0.25 g of Vitamin B1
0.3 g of Vitamin B2
an appropriate amount of purified water

The above ingredients were mixed according to a conventional method for manufacturing health drinks, stirred and heated at 85 °C for about 1 hour, and the resulting solution was filtered. The filtrate was filled into a sterilized 2 L container, sealed, sterilized, and stored in a refrigerator. The resulting solution may be used to manufacture a health functional food composition (e.g., in beverage form).

The composition ratio described above represents a preferred example of a mixture of ingredients suitable for beverages. However, the mixing ratio may be arbitrarily modified according to regional or ethnic preferences, such as consumer groups, countries, or intended uses.

[Industrial Applicability]

The present invention exhibits the effect of effectively treating, preventing, improving, and/or suppressing one or more conditions selected from obesity and obesity-related complications. Accordingly, the present invention has industrial applicability.

## Claims

1. A pharmaceutical composition for the treatment or prevention of obesity, comprising a peptide having an amino acid sequence of SEQ ID NO: 1 or a pharmaceutically acceptable salt thereof as an active ingredient.

2. A food composition for the improvement or prevention of obesity, comprising a peptide having an amino acid sequence of SEQ ID NO: 1 or a pharmaceutically acceptable salt thereof as an active ingredient.

3. The food composition according to claim 2, wherein the food composition is a health functional food composition.

4. A method for treating, preventing, improving, or suppressing obesity, comprising administering a peptide having an amino acid sequence of SEQ ID NO: 1 or a pharmaceutically acceptable salt thereof to a non-human animal.

[FIG. 1]

[FIG. 2]

## INTERNATIONAL SEARCH REPORT

| | International application No. |
|---|---|
| | **PCT/KR2024/012000** |

**A.   CLASSIFICATION OF SUBJECT MATTER**

**A61K 38/08**(2006.01)i; **A61P 3/04**(2006.01)i; **A23L 33/18**(2016.01)i

According to International Patent Classification (IPC) or to both national classification and IPC

**B.   FIELDS SEARCHED**

Minimum documentation searched (classification system followed by classification symbols)

A61K 38/08(2006.01); A23K 10/22(2016.01); A23K 20/147(2016.01); A61K 38/00(2006.01); C07K 14/47(2006.01); C07K 7/06(2006.01); C07K 7/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 펩타이드(peptide), 비만(obesity), 투여(administration), 티로신(tyrosine), 글라이신(glycine), 알라닌(alanine)

**C.   DOCUMENTS CONSIDERED TO BE RELEVANT**

| Category* | Citation of document, with indication, where appropriate, of the relevant passages | Relevant to claim No. |
|---|---|---|
| A | KR 10-1995768 B1 (ENSOL BIOSCIENCES INC.) 03 July 2019 (2019-07-03)<br>    See paragraph [0009]; and claims 1, 2 and 4. | 1-4 |
| A | KR 10-2021-0052360 A (KYONGSANGNAM-DO) 10 May 2021 (2021-05-10)<br>    See claims 1, 3 and 5. | 1-4 |
| A | KR 10-2018-0043428 A (CAREGEN CO., LTD.) 30 April 2018 (2018-04-30)<br>    See claims 1, 2 and 12. | 1-4 |
| A | KR 10-2022-0090473 A (MEDI&GENE) 29 June 2022 (2022-06-29)<br>    See claims 1-3. | 1-4 |
| A | KR 10-2426869 B1 (MEDINUTRA CO., LTD. et al.) 29 July 2022 (2022-07-29)<br>    See claims 1, 2 and 11. | 1-4 |

☑ Further documents are listed in the continuation of Box C.     ☑ See patent family annex.

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 November 2024** | **20 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/012000**

| C. | DOCUMENTS CONSIDERED TO BE RELEVANT | |
|---|---|---|
| Category* | Citation of document, with indication, where appropriate, of the relevant passages | Relevant to claim No. |
| PX | KR 10-2624902 B1 (ENSOL BIOSCIENCES INC.) 17 January 2024 (2024-01-17) See claims 1-4. This document is a published earlier application that serves as a basis for claiming priority of the present international application. | 1-4 |

Form PCT/ISA/210 (second sheet) (July 2022)

**INTERNATIONAL SEARCH REPORT**

International application No.

**PCT/KR2024/012000**

| Box No. I | Nucleotide and/or amino acid sequence(s) (Continuation of item 1.c of the first sheet) |

1.  With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/KR2024/012000**

| Patent document cited in search report | | | Publication date (day/month/year) | Patent family member(s) | | | Publication date (day/month/year) |
|---|---|---|---|---|---|---|---|
| KR | 10-1995768 | B1 | 03 July 2019 | CN | 113508126 | A | 15 October 2021 |
| | | | | CN | 113508126 | B | 29 September 2023 |
| | | | | EP | 3938376 | A1 | 19 January 2022 |
| | | | | JP | 2022-524585 | A | 09 May 2022 |
| | | | | JP | 7163547 | B2 | 01 November 2022 |
| | | | | US | 2022-0135621 | A1 | 05 May 2022 |
| | | | | WO | 2020-184901 | A1 | 17 September 2020 |
| KR | 10-2021-0052360 | A | 10 May 2021 | KR | 10-2021-0052381 | A | 10 May 2021 |
| | | | | KR | 10-2021-0052382 | A | 10 May 2021 |
| | | | | KR | 10-2288716 | B1 | 12 August 2021 |
| | | | | KR | 10-2537907 | B1 | 31 May 2023 |
| | | | | KR | 10-2537908 | B1 | 31 May 2023 |
| KR | 10-2018-0043428 | A | 30 April 2018 | CN | 110088122 | A | 02 August 2019 |
| | | | | CN | 110088122 | B | 27 December 2022 |
| | | | | EP | 3530667 | A1 | 28 August 2019 |
| | | | | EP | 3530667 | B1 | 17 November 2021 |
| | | | | JP | 2019-533680 | A | 21 November 2019 |
| | | | | JP | 6875516 | B2 | 26 May 2021 |
| | | | | KR | 10-1887577 | B1 | 10 September 2018 |
| | | | | US | 10723777 | B2 | 28 July 2020 |
| | | | | US | 2020-0048323 | A1 | 13 February 2020 |
| | | | | WO | 2018-074682 | A1 | 26 April 2018 |
| KR | 10-2022-0090473 | A | 29 June 2022 | CN | 116888145 | A | 13 October 2023 |
| | | | | EP | 4269428 | A1 | 01 November 2023 |
| | | | | JP | 2024-503540 | A | 25 January 2024 |
| | | | | US | 2024-0034758 | A1 | 01 February 2024 |
| | | | | WO | 2022-139487 | A1 | 30 June 2022 |
| KR | 10-2426869 | B1 | 29 July 2022 | None | | | |
| KR | 10-2624902 | B1 | 17 January 2024 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020184901 A1

### Non-patent literature cited in the description

- **POWELL-WILEY TM** ; **POIRIER P** ; **BURKE LE** ; **DESPRÉS JP** ; **GORDON-LARSEN P** ; **LAVIE CJ** ; **LEAR SA** ; **NDUMELE CE** ; **NEELAND IJ** ; **SANDERS P**. Obesity and cardiovascular disease: A Scientific statement from the American Heart Association. *Circulation*, 25 May 2021, vol. 143 (21), e984-e1010
- **BLÜHER M**. Obesity: global epidemiology and pathogenesis. *Nat Rev Endocrinol.*, May 2019, vol. 15 (5), 288-298
- **WHITLOCK G** ; **LEWINGTON S** ; **SHERLIKER P** ; **CLARKE R** ; **EMBERSON J** ; **HALSEY J** ; **QIZILBASH N** ; **COLLINS R** ; **PETO R**. Body-mass index and cause-specific mortality in 900,000 adults: Collaborative Analyses of 57 Prospective Studies. *Lancet*, 28 March 2009, vol. 373 (9669), 1083-96
- **KARAMPATSI D** ; **ZABALA A** ; **WILHELMSSON U** ; **DEKENS D** ; **VERCALSTEREN E** ; **LARSSON M** ; **NYSTRÖM T** ; **PEKNY M** ; **PATRONE C** ; **DARSALIA V**. Diet-induced weight loss in obese/diabetic mice normalizes glucose metabolism and promotes functional recovery after stroke. *Cardiovasc Diabetol*, 22 December 2021, vol. 20 (1), 240
- **REISIN E** ; **ABEL R** ; **MODAN M** ; **SILVERBERG DS** ; **ELIAHOU HE** ; **MODAN B**. Effect of weight loss without salt restriction on the reduction of blood pressure in overweight hypertensive patients. *N Engl J Med*, 05 January 1978, vol. 298 (1), 1-6
- **POWELL-WILEY TM** ; **POIRIER P** ; **BURKE LE** ; **DESPRES JP** ; **GORDON-LARSEN P** ; **LAVIE CJ** ; **LEAR SA** ; **NDUMELE CE** ; **NEELAND IJ** ; **SANDERS P**. Obesity and cardiovascular disease: A scientific statement from the American Heart Association. *Circulation*, 25 May 2021, vol. 143 (21), e984-e1010
- **WHITLOCK G** ; **LEWINGTON S** ; **SHERLIKER P** ; **CLARKE R** ; **EMBERSON J** ; **HALSEY J** ; **QIZILBASH N** ; **COLLINS R** ; **PETO R**. Body-mass index and cause-specific mortality in 900,000 adults: collaborative analyses of 57 prospective studies. *Lancet*, 28 March 2009, vol. 373 (9669), 1083-96
- USFDA, Guidance for Industry: Estimating the Maximum Safe Starting Dose in Adult Healthy Volunteers. US Food and Drug Administration, 2005